Europäisches Patentamt

European Patent Office

Office européen des brevets

(19)

(11) Publication number: **0 265 858**
**A1**

(12) **EUROPEAN PATENT APPLICATION**

(21) Application number: 87115542.0

(51) Int. Cl.⁴: **A61K 31/405**

(22) Date of filing: 23.10.87

(30) Priority: 29.10.86 IT 2217186
29.10.86 IT 2217286

(43) Date of publication of application:
04.05.88 Bulletin 88/18

(84) Designated Contracting States:
AT CH DE FR GB IT LI LU NL SE

(71) Applicant: "IBIS" ISTITUTO BIOCHIMICO
SPERIMENTALE S.p.A.
Viale Macchiavelli, 31
I-50125 Firenze(IT)

(72) Inventor: Tonozzi, Maria Alessandra
Viale Machiavelli, 31
Firenze(IT)
Inventor: Mazzoni, Lucia
Viale Machiavelli, 31
Firenze(IT)

(74) Representative: Minoja, Fabrizio
Studio Consulenza Brevettuale Via Rossini,
8
I-20122 Milano(IT)

(54) Topical antiinflammatory compositions.

(57) Topical pharmaceutical compositions for dermatological or ophthalmic use containing acemetacin as the active ingredient are described.

The compositions of the invention are useful in the treatment of phlogistic or inflammatory conditions of various localization.

EP 0 265 858 A1

## TOPICAL ANTIINFLAMMATORY COMPOSITIONS.

The present invention relates to topical antiinflammatory compositions containing 1-(4-chlorobenzoyl)-5-methoxy-2-methyl-1H-indole-3-acetic acid, known as acemetacin, as the active ingredient.

Acemetacin, which is disclosed in German Patent No. 2 234 651, is a well-known antiinflammatory agent, and it has already been used in human therapy, due its pharmacological activities (Arzn. Forsch. 30, 1398, 1980). The only administration route hitherto known is the oral one. Now it has been found that administration of acemetacin by the topical route, either dermatological or ophtalmological in form of suitable formulations, is well tolerated and it allows to attain advantageous therapeutic effects. Pathological conditions which may be advantageously treated with dermatological compositions according to the invention comprise, for instance, reumathoid arthritis, inflammatory diseases of various localization (tendinitis, tenosynovitis, bursitis, thrombophlebitis, distortions, hematomas, myalgia, lumbagos, periarthritis, bruises, sprains, oedemas, and traumatic infiltrations).

Ophthalmic pathologies which may be advantageously treated with the ophthalmic compositions of the invention comprise, for instance, uveitis, oedema of the cornea, scleritis, irites, oedema of the conjunctiva, ocular wounds, conjunctivitis, pre- and post-surgical treatment of the cataract, corneal neo-vascularization by contact lenses.

Particularly, it has been found that topical administration is extremely advantageous in comparison with oral administration, undesired side-effects being remarkably lower. Moreover, very high concentrations of the active ingredient in synovial fluid or in the conjunctiva are quickly attained after topical administration.

The topical compositions of the invention may contain the active ingredient in amounts from 0.1 to 5% by weight, more preferably 0.5 to 2% by weight, suitably formulated using pharmaceutically acceptable carriers or excipients.

Examples of said carriers and excipients for dermatological compositions comprise oily excipients, such as fats, vegetal oils, cocoa butter, carnauba wax, spermacetis, vaselin, silicons; adsorption excipients such as lanolin, higher fatty acids; emulsifying and/or hydrosoluble excipients, such as glycerin, propylene glycol, polyethylene glycol, cellulose derivatives, tragacanth gum, pectin, as well as antibacterial agents, preservatives, perfumes, flavors, etc.

For ophthalmic applications, suitable formulation are sterile solutions or ointments, preferably isotonic, suitably prepared using well-known carriers such as water or mixture of hydrosoluble solvents, e.g. lower alcohols polyalkylene glycols, carboxymethylcellulose, polyvinylpyrrolidone, isopropylmiristate, polysorbate and the like, as well as buffering, anti-bacterial, emulsifying, surfactant agents and salts (sodium chloride, sodium borate) in order to make the solution isotonic.

As a buffering agent, an usual phosphate buffer is normally used whereas as preservative and anti-bacterial agent a quaternary ammonium salt such as benzalkonium chloride or the like, thimerosal, methyl and propyl paraben, benzyl alcohol, phenylethanol, phenylmercuric acetate, etc. may be suitably used. Acemetacin may also be used in form of a non-toxic, pharmaceutically acceptable salt for the formulation of collyriums or eye-drops according to the invention.

The excipients will be selected in view of the intended formulation, according to commonly available knowledges, as reported for example in "Remington's Pharmaceutical Sciences Handbook", Hack Publishing Co., U.S.A.

It is moreover obvious that other substances may be used in addition to or instead of the above mentioned ones, as it is well-known to the skilled in the art.

All of these formulations are of course within the spirit and scope of the invention, even though they are not specifically described. The preparation of the pharmaceutical compositions above described is also carried out according to conventional techniques, for example by mechanical incorporation of the active ingredient in the excipient base, by melting, by emulsyfication, by one or more sterilization steps through sterilizing filters, etc.

The compositions according to the invention may optionally contain other active ingredient, having complementar or anyhow useful activities, such as antiseptics, analgesics, heparinoids, or, in the ophthalmic formulations, antibiotics, vitamins, amino acids and the like.

The following non-limiting examples further illustrate the present invention.

EXAMPLE 1

2% Cream
Composition100 g of cream contain:
Acemetacin     2 g
Excipients:

| | |
|---|---|
| Fatty acid polyglycolic esters | 18 g |
| Propylene glycol | 7 g |
| Cetostearyl alcohol | 3.5 g |
| 1000 monocetylether polyethylene glycol | 2 g |
| Phenylethyl alcohol | 0.5 g |
| Sodium citrate | 0.402 g |
| Citric acid monohydrate | 0.28 g |
| Anallergenic perfume | 0.1 g |
| Depurated water q. s. to | 100 g |

EXAMPLE 2

2% Gel

| | |
|---|---|
| CompositionAcemetacin | 2 g |
| Excipients: | |
| Carbopol 940 | 0.8 g |
| Triethanolamine | 1.08 g |

EXAMPLE 3

1% Collyrium CompositionAcemetacin

| | |
|---|---|
| | 1 g |
| Polysorbate 80 | 0.10 g |
| Benzalkonium chloride | 0.02 g |
| Hydroxypropylmetylcellulose | 0.6 g |
| Disodium phosphate | 0.10 g |
| Sodium chloride | 5 g |
| Sodium edetate | 0.01 g |
| Distilled water q.s. to | 100 ml |

Pharmaco-toxicological tests showed the high efficacy and the complete lack of irritative or allergic phenomena of the above formulation.

The collyrium will be usually administered 1,2 or more times a day, in a dose of 1 or 2 drops, according to the kind and to the seriousness of the disease to be treated.

**Claims**

1. Topical pharmaceutical compositions having antiinflammatory activity, containing acemetacin as the active ingredient, together with an appropriate pharmaceutical carrier.

2. Pharmaceutical compositions according to claim 1, wherein the active ingredient is present in amounts from 0.1 to 5% by weight.

3. Pharmaceutical compositions according to claim 1 or 2 for the dermatological use.

4. Pharmaceutical compositions according to claim 3, in form of creams, gels, ointments, pastes, unguents.

5. Pharmaceutical compositions according to claim 1 or 2 for ophthalmic use.

6. Pharmaceutical compositions according to claim 5, in form of sterile solution or ointment.

European Patent Office

**EUROPEAN SEARCH REPORT**

Application Number

EP 87 11 5542

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 148 391 (BAYER AG)<br>* Page 30, example 14; page 31, claim 1 * | 1-4 | A 61 K 31/405 |
| X | US-A-3 966 956 (BOLTZE et al.)<br>* Column 1, lines 8-40; column 7, lines 3-4,45-47 * & DE-A-2 234 651 (Cat. D,X) | 1-4 | |
| A | EP-A-0 055 029 (KOWA CO. LTD) | | |
| X,Y | DE-A-3 026 402 (SYNTEX CORP.)<br>* Claim 1; in particular page 1, lines 29,30; page 10, lines 16-20 * | 5,6 | |
| Y | US-A-4 087 538 (PORTNOFF)<br>* Whole document * | 5,6 | |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 22-12-1987 | BENZ K.F. |